# EUROPÄISCHE PATENTSCHRIFT

(11) **EP 3 664 757 B1**
(45) Veröffentlichungstag und Bekanntmachung des Hinweises auf die Patenterteilung: **01.09.2021**
(21) Anmeldenummer: 18749326.7
(22) Anmeldetag: 26.07.2018
(51) Int. Cl.: A61F 13/02, A61F 13/00

(54) **BINDE, INSBESONDERE KOMPRESSIONSBINDE**
BANDAGE, IN PARTICLAR A COMPRESSION BANDAGE
BANDAGE, EN PARTICULIERS BANDAGE DE COMPRESSION

(30) Priorität: 07.08.2017 DE 102017117828
(43) Veröffentlichungstag der Anmeldung: 17.06.2020
(73) Patentinhaber: IVF Hartmann AG, 8212 Neuhausen am Rheinfall (CH)
(72) Erfinder: BERNET, Claudia, 8460 Marthalen (CH); HOESL, Tobias, 79787 Lauchringen (DE); ROTHMAIER, Markus, 8308 Illnau (CH)
(74) Vertreter: DREISS Patentanwälte PartG mbB
(86) Internationale Anmeldenummer: PCT/EP2018/070332
(87) Internationale Veröffentlichungsnummer: WO 2019/030011

(56) Entgegenhaltungen:
- WO-A1-97/29689
- DE-A1- 2 515 786
- US-A1- 2007 185 428

## Beschreibung

Die Erfindung betrifft eine Binde, insbesondere eine Kompressionsbinde, zur Erstversorgung von Verletzungen. Im Stand der Technik sind vielfache Binden und Bandagen bekannt, wobei die Begriffe "Binden" und "Bandagen" im Folgenden synonym verwendet werden sollen, die zu verschiedenen Einsatzzwecken Verwendung finden. Dabei sind Binden bekannt, die bei der Erstversorgung von Verletzungen eingesetzt werden sollen, wobei es sich häufig um durch Traumata entstandene Verletzungen und Wunden handelt, die häufig stark blutend sind. Für solche Einsatzzwecke, die auch als Krisensituationen beschrieben werden können, ist es bekannt, Binden einzusetzen, die zum einen ein Wundkissen aufweisen, das auf einer Seite der Flachmaterialbahn, aus der die Binde als solche besteht, aufgebracht ist, und auf der anderen Seite ein Druckapplikator vorgesehen ist, der das Aufbringen einer hinreichenden Kompression auf die Wunde ermöglicht.

Gerade bei stark blutenden Wunden ist es wichtig, einen hinreichenden Druck aufzubringen, um einen zu starken Blutverlust zu vermeiden.

So ist aus dem Stand der Technik beispielsweise die WO 97/29689 A1 bekannt, die eine Bandage mit einem sterilen Wundkissen beschreibt, wobei auf der Rückseite gegenüberliegend zum Wundkissen ein Druckapplikator vorgesehen ist, durch den die Bandage hindurchgezogen wird, und der dann einen erhöhten Druck auf eine darunter liegende Verletzung aufbringt. Nachteilig bei der Gestaltung ist dabei, dass der aufgebrachte Druck auf eine relativ kleine Fläche appliziert wird und eine inhomogene Druckverteilung besteht.

Weiterhin ist beispielsweise aus der US 2003/199801 A1 eine Gestaltung bekannt, bei der auf der Rückseite der Bandage ein hakenförmiges Element vorgesehen ist. Nachdem die erste Wicklung der Binde aufgebracht ist und diese an einem Körperteil fixiert ist, wird dann bei der nächsten Wicklung die Binde durch den Haken durchgeführt wird, und danach die Wicklungsrichtung geändert. Hierdurch können Zugkräfte aufgebracht werden, die über die Mitte des Wundkissens in die beteiligte Körperregion eingeleitet werden und dadurch einen gleichmäßigeren Druck ausüben sollen. Nachteilig ist hierbei, dass durch die einseitigen Zugkräfte es zu einem Verschieben der Bandage kommen kann.

Eine weitere Verbesserung zeigt nun die US 2007/0185428 A1. Diese zeigt mittig über dem Wundkissen ein H-förmiges Element zur Aufbringung von Druck auf eine Verletzung. Dabei wird die Bandage nach einer ersten Fixierung weiter um die Gliedmaße gewickelt, um dann um ein Seitenteil des H-förmigen Elements herumgeschlungen zu werden und so einen Richtungswechsel zu vollführen, wodurch ein erster Zug aufgebracht werden kann. Bei Wiederreichen des H-förmigen Elements wird dann die Binde wiederum um das zweite Seitenteil des H herumgeführt, um eine erneute Richtungsänderung herbeizuführen. Es ist nun ein gleichmäßigeres Aufbringen von Zugkräften möglich.

Nachteilig bei der vorliegenden Gestaltung ist, dass durch das Herumführen um das H-förmige Element es zu einer Verkleinerung der Auflagefläche der Binde in diesen Bereich kommt und es dann erst wieder eine gewisse Strecke benötigt, bis die Binde wieder über ihre volle Breite aufgespreizt wird. Darüber hinaus ist das H-förmige Element vergleichsweise klein hinsichtlich der Fläche, so dass der aufgebrachte Druck durch das Spannen der Bandage eher gering ist und nicht besonders flächig ist.

Die Erfindung stellt sich nun die Aufgabe, eine Binde bereitzustellen, die eine einfache Applizierbarkeit und Versorgung von Verletzungen, insbesondere auch in Krisensituationen, ermöglicht, wobei in Krisensituationen eine einfache Handhabbarkeit besonders wichtig ist.

Die Erfindung löst diese Aufgabe durch eine Binde mit folgenden Merkmalen des Anspruchs 1, nämlich einer Binde umfassend
a. eine Flachmaterialbahn mit einer ersten und einer zweiten Seite und einem ersten und zweiten in Längsrichtung der Binde beabstandeten Ende,
b. ein Wundkissen auf der ersten Seite der Flachmaterialbahn,
c. einen Druckapplikator auf der zweiten Seite der Flachmaterialbahn gegenüber dem Wundkissen,
d. ein Befestigungselement am zweiten Ende der Flachmaterialbahn zur Befestigung des zweiten Endes der Flachmaterialbahn auf oder an sich selber bei an einem Träger angelegter Binde,
wobei der Druckapplikator eine Basisplatte aufweist und zwei in Längsrichtung der Binde beidseits der Basisplatte angeordnete längliche Fixierungselemente, die sich in einem Winkel, insbesondere senkrecht, zur Längsrichtung der Binde erstrecken, wobei jeweils das eine Ende der Fixierungselement an der Basis festgelegt ist und das andere Ende frei ist und die Fixierungselemente über ihre restliche Länge zwischen sich und der Basisplatte einen Spalt einschließen.

Durch die Vorsehung von zwei länglichen Fixierungselementen in Längsrichtung beidseits einer Basisplatte wird erreicht, dass beim Anlegen der Binde analog zum Stand der Technik die Binde zwischen der Basisplatte und den Fixierungselementen hindurchgeführt werden kann, um dann bezüglich der Umwicklungsrichtung des Körperteils eine Richtungsänderung zu vollführen und dann weiter um den Körperteil herumgeschlungen zu werden, bis das zweite Fixierungselement erreicht wird, wobei die Binde dann wiederum zwischen Fixierungselement und Basisplatte hindurchgeführt wird und wiederum eine Richtungsänderung des Anlegens erfolgt, so dass durch Anziehen der Binde ein gleichmäßiger in beide Richtungen (Bindenlängsrichtung) wirkender Druck durch die Basisplatte auf eine Verletzung aufgebracht werden kann.

Dabei ist die Vorsehung von länglichen Fixierungselementen besonders vorteilhaft, da es nicht zu einem Einschnüren der Bindenbreite beim Herumlegen der Binde um den Druckapplikator kommt. Vielmehr kann der über den Zug aufgebrachte Druck auf die Basisplatte auf eine Verletzung über die gesamte Bindenbreite sehr gleichmäßig erzeugt werden. Dabei weist der Druckapplikator eine im Wesentlichen W- oder S-förmige Form auf bestehend aus einer Basisplatte, die jeweils beidseits in Bindenlängsrichtung mit länglichen Fixierungselementen verbunden ist. Eine bevorzugte S-förmige Gestaltung ergibt sich, sofern die an der Basisplatte festgelegten Enden der Fixierungselemente einander diagonal gegenüberliegen. Hierdurch kann das Aufbringen des Drucks noch gleichmäßiger erfolgen und ein ungewolltes Herausrutschen der Binde aus dem Druckapplikator wird besser vermieden. Sind die Fixierungselemente auf der gleichen Seite der Basisplatte festgelegt, ergibt sich die W-förmige Anmutung.

Durch die Ausgestaltung des Druckapplikators ergibt sich das Anlegen der Binde für einen Benutzer annähernd intuitiv.

Dabei ist vorgesehen, dass die Binde zunächst mit ihrem Wundkissen auf eine Verletzung aufgebracht wird und dann so weit abgerollt und um eine Gliedmaße oder einen sonstigen Körperteil herumgelegt wird, bis die Bandage oder Binde in Eingriff mit dem Druckapplikator gebracht werden kann. Die Binde wird dann in den Spalt zwischen erstem Fixierungselement und Basisplatte eingeführt und es erfolgt dann ein Richtungswechsel der Anlegerichtung, wodurch ein erster Zug aufgebracht werden kann und gleichzeitig das erste Ende fixiert ist. Dabei ist es auch möglich, jetzt noch die Position des Wundkissens bezüglich einer Verletzung zu justieren. Die Binde wird dann so weit um den betroffenen Körperteil herumgeschlungen, bis die Binde wieder in den Bereich des Druckapplikators kommt und wird in den Spalt zwischen dem zweiten Fixierungselement und der Grundplatte eingeführt. Wird nun an der Binde gezogen, so kann der gewünschte Druck aufgebracht werden, wobei sich die Druckverteilung durch die zweifache Umlenkung der Binde gleichmäßig über den Druckapplikator auf die Verletzung verteilt. Die Binde kann anschließend um die betroffene Stelle herumgelegt werden, wodurch ein zusätzlicher Druck über den Druckapplikator senkrecht in die Verletzung eingeleitet wird. Dieser aufgebrachte Druck ergibt sich stets, wenn ein zusätzliches Element unter einer entsprechenden Bandage eingewickelt wird. Ist die Binde dann bezüglich ihrer vollen Länge am Träger angebracht, kann sie mittels des Befestigungselements, das am zweiten Ende der Flachmaterialbahn vorgesehen ist, beispielsweise auf oder an sich selber festgelegt werden.

Dabei sind verschiedene Befestigungselemente denkbar. So kann beispielsweise ein Haken- und Schlaufenelement (Velcro-Befestigungselement) vorgesehen sein, das am Bindenmaterial festgelegt werden kann. Darüber hinaus ist auch ein bügelförmiges Verschlusselement denkbar, wobei die freien Enden der Bügel unter Wicklungen der Bandage gehakt werden können. Alternativ sind sämtliche weitere aus dem Binden- und Bandagenbereich bekannte Befestigungselemente möglich. Unter "Befestigungselement" soll dabei auch verstanden werden, dass das zweite Ende der Binde selber das Befestigungselement bildet, indem dieses beispielsweise unter andere Wicklungen der Binde heruntergesteckt wird und so über Reibung festgelegt ist. Darüber hinaus soll hierunter auch verstanden werden, wenn die Befestigung der Binde am Träger über externe Elemente, wie Klammern, etc., erfolgt, die mit dem zweiten Ende, das dann das zweite Befestigungselement bildet, zusammenwirken. Bei der Vorsehung eines bügelförmigen Befestigungselementes kann dieses auch zum Abbinden der Gliedmaße eingesetzt werden, in dem das bügelförmige Verschlusselement unter darunter liegenden Wicklungen der Binde hindurchgeführt wird und um eine Achse senkrecht zur Bindenebene gedreht wird und so der Druck auf eine Wunde erhöht wird. Anschließend wird das bügelförmige Verschlusselement in herkömmlicher Weise mit seinen Enden unter darunter liegende Wicklungen eingehakt und gegen Entdrehen fixiert.

Dabei ist es besonders bevorzugt, wenn die Basisplatte eine rechteckige oder parallelogrammartige Form aufweist. Hierdurch kann eine vergleichsweise großflächige und gleichmäßige Krafteinleitung erfolgen und insbesondere können die Fixierungselemente parallel zu den Längsseiten des Rechtecks oder Parallelgramms vorgesehen sein, das sich über einen gewünschten Abschnitt der Breite der Binde erstrecken kann. Darüber hinaus kann die Basisplatte flach oder alternativ auch gekrümmt ausgebildet sein, um eine anatomische Anpassung an einen Körperteil vorzusehen. Ggf. kann sie auch flexibel gestaltet sein, so dass sich die Basisplatte in gewissem Rahmen an einen Körperteil anpassen lässt.

Um ein besonders einfaches Applizieren und insbesondere Einführen der Binde in den Spalt zu ermöglichen, kann vorgesehen sein, dass sich die Fixierungselemente insbesondere quer zur Längsrichtung der Binde über die Basisplatte hinaus erstrecken. Dabei ist es aus Gründen der flächigen Aufbringung der Zugkräfte besonders wünschenswert, wenn die Spalte senkrecht, also quer zur Bindenlängsrichtung, verlaufen, da dann eine besonders gleichmäßige Zugkraft erreicht werden kann und gleichzeitig es nicht zu einem Einschnüren der Bindenbreite, also einer Verringerung der Bindenbreite, mehr als notwendig, kommt.

Dabei ist es besonders bevorzugt, wenn der Spalt zwischen den Fixierungselementen der Basisplatte im Wesentlichen gerade verläuft, da auch dies den Bindenverlauf bei der Umlenkung positiv beeinflusst. Besonders einfach wird das Einführen der Binde zwischen den Fixierungselementen und der Basisplatte, wenn, wie es insbesondere bevorzugt vorgesehen ist, die Fixierungselemente und/oder die Basisplatte wenigstens abschnittsweise elastisch verformbar sind. Der Spalt kann dabei über seine Länge die gleiche Breite aufweisen oder sich verjüngen oder verbreitern. Weiterhin kann, um ein Herausrutschen der Binde aus dem Spalte zu verhindern, vorgesehen sein, an den freien Enden der Fixierungselemente in den Spalt hineinragende und diesen gegebenenfalls überspannende und damit gegen die Basisplatte anliegende Nasen anzuordnen.

Der Druckapplikator ist dabei vorzugsweise mittig über dem Wundkissen auf der gegenüberliegenden Seite der Flachmaterialbahn angeordnet. Hierdurch kann der Druck besonders zentral eingebracht werden und eine Positionierung des Wundkissens auf einer Verletzung ist besonders einfach möglich.

Um eine einfache Positionierbarkeit weiter zu verbessern und die Applikation zu erleichtern, kann vorgesehen sein, dass die Flachmaterialbahn einen Anfassabschnitt am ersten Ende aufweist. Dabei ist das erste Ende das Ende, das bei einer in Rollenform konfigurierten Binde außen liegend ist und das zunächst ergriffen wird. Um hier eine bessere Händelbarkeit zu erreichen, kann der Anfassabschnitt dadurch gebildet werden, dass die Flachmaterialbahn auf sich selber umgeschlagen und festgelegt wird. Dieser Anfassabschnitt, der einen doppellagigen endständigen Bereich bildet und der insbesondere durch Nähen fixiert werden kann, kann in etwa eine Länge von 5 bis 15 cm, vorzugsweise von 7 bis 12 cm, und weiter vorzugsweise von ca. 5 cm bis 10 cm aufweisen. Hierdurch wird das Handling besonders vereinfacht. Auf der Innenseite der Binde schließt sich dann vorzugsweise das Wundkissen an.

Dabei kann das Wundkissen vorzugsweise absorbierend ausgestaltet sein und insbesondere aus einem Hüllmaterial bestehen, wobei die Umhüllung auf der wund- oder verletzungszugewandten Seite aus einem flüssigkeitsdurchlässigen, hydrophilen Vlies bestehen kann, das insbesondere aus Polypropylen oder einer Mischung aus Polyamid und Viskose (55/45) hergestellt ist. Auf der wundabgewandten und damit der Binde zugewandten Seite kann ein hydrophobes Vliesmaterial aus, vorzugsweise aus Polypropylen, vorgesehen sein. Die Hülllagen können aber auch identisch ausgebildet sein. Die beiden Hülllagen können beispielsweise durch Ultraschallverschweißung, aber auch durch jedes beliebige herkömmliche Verbindungsverfahren miteinander verbunden sein. Das absorbierende Material ist dabei vorzugsweise innerhalb der Umhüllung vorgesehen. Hierbei kann es sich beispielsweise um einen Kern aus Zellulosefasern in wenig verfestigter Lage (Fluff) handeln. Dabei können weitere oder zusätzliche Materialien das absorbierende Material bilden oder darin vorgesehen sein, wie z.B. Alginat, SAP, blutstillende oder infektionshemmende Materialien.

Besonders bevorzugt kann dabei vorgesehen sein, wenn eine weitere innere Umhüllung vorgesehen ist, die beispielsweise durch ein dünnes Zellulosepapier gebildet werden kann. Das absorbierende Material, insbesondere das Fluffmaterial, kann vorzugsweise in der inneren Umhüllung vorgesehen sein. Diese ist dann in dem äußeren Hüllmaterial aufgenommen.

Besonders bevorzugt ist es dabei, wenn die Breite der Wundauflage der Breite der Binde entspricht. Bevorzugte Bindenbreiten sind 10 cm, 15 cm oder 20 cm. So kann beispielsweise vorgesehen sein, je nach Bindenbreite ein Wundkissen in der Größe von 5 x 5 cm, aber auch bis 20 x 40 cm vorzusehen. Besonders bevorzugt sind dabei Wundkissen in der Größe 10 x 20 cm, 15 x 20 cm oder 20 x 20 cm. Neben derartigen rechteckigen Wundkissen sind auch quadratische oder runde Wundkissen denkbar. Die Wundkissen können mit der Bandage vernäht oder verklebt oder sonstwie an dieser befestigt sein. Als Wundkissen kommt beispielsweise das Produkt Zetuvit® der Paul Hartmann AG, Heidenheim, Deutschland, in Betracht.

Bei der Flachmaterialbahn kann es sich vorzugsweise um ein monoelastisches Gewebe handeln, es ist dabei weiterhin besonders bevorzugt, wenn die Binde als Kompressionsbinde gestaltet ist. Dabei ist es besonders bevorzugt, wenn die Elastizität durch Einsetzen elastischer Kettfäden erzielt wird. Im ungedehnten Zustand weist eine solche Binde eine wellenartige Oberfläche auf. Die Elastizität derartiger Binden wird in der Regel dadurch erreicht, dass die elastischen Fasern, die als Kettfäden eingesetzt werden, beim Webvorgang vorgespannt sind und dann zur Konfektionierung entspannt werden, so dass sich eine wellenartige Oberfläche ausbildet. Besonders bevorzugt ist dabei, wenn die Binde eine Elastizität in Längsrichtung von nicht mehr als 250%, insbesondere von nicht mehr als 200% und weiterhin von 160-200%, vorzugsweise von 175% oder 200% aufweist.

Die Elastizität der Binde ist dabei nach DIN 61632:2009-12 bestimmbar.

Der Druckapplikator ist vorzugsweise aus Kunststoff und kann besonders bevorzugt aus Polycarbonat bestehen. Generell sind jedoch auch andere Kunststoffe verwendbar. Dabei weist der Druckapplikator vorzugsweise Abmessungen von in Bindenlängsrichtung von 50-80 mm, vorzugsweise 60-70 mm auf. Quer zur Bindenlängsrichtung ist die Erstreckung vorzugsweise 5-15 mm kleiner als die Bindenbreite, vorzugsweise 10 mm kleiner als die Bindenbreite.

Besonders bevorzugt ist dabei, wenn die Kompressionsbinde in Rollenform konfektionierbar ist, so dass das erste Ende der Flachmaterialbahn außen liegend ist.

Dabei ist es weiterhin besonders bevorzugt, wenn ein Abrollen eines ersten Bereichs einhändig möglich ist und ein weiteres Abrollen dann zunächst durch eine Abrollsicherung verhindert wird. Dieser erste Bereich kann vorzugsweise weniger als 60 cm, vorzugsweise weniger als 50 cm und vorzugsweise 35 cm bis 45 cm betragen. Hierdurch wird das Handling der Binde verbessert, da sich der Anlegende auf das Wundkissen in dieser ersten Phase konzentrieren kann und nicht darauf achten muss, dass er die Bandage sicher hält und es nicht zu einem ungewollten Abwickeln der Bandage kommt.

Es kann zusätzlich vorgesehen sein, wie es auch im Stand der Technik bekannt ist, auch im Bereich des ersten Endes ein Haftelement entweder mechanisch oder adhäsiv oder kohäsiv vorzusehen, um die erste Wicklung zu sichern.

Grundsätzlich ist jedoch auch die Sicherung der ersten Wicklung auf sich selber allein durch die Reibung der Lagen aufeinander als ausreichend anzusehen.

Zum weiteren Anlegen der Binde ist es - wenn eine Abrollsicherung vorgesehen ist - dann erforderlich, dass die Abrollsicherung aufgehoben wird. Dazu kann vorgesehen sein, dass die Abrollsicherung lösbar und/oder zerstörbar ist. Beispielsweise kann als Abrollsicherung vorgesehen sein, dass ein Faden vorgesehen ist, der die oberste Lage der Bindenrolle mit darunter liegenden Lagen verbindet und so ein weiteres Abrollen ab der mit der Abrollsicherung versehenen Stelle der Binde verhindert. Durch einen kräftigeren Zug kann dann der Faden zerstört werden, so dass das vollständige Abrollen der Binde und damit das Anlegen derselben an einem Patienten möglich wird.

Das Anlegen der Binde erfolgt dabei nun wie folgt: Zur Anwendung der Binde wird diese aus einer Verpackung entnommen, wobei es vorzugsweise vorgesehen ist, dass die Binde steril konfektioniert sein kann. Das erste Ende der Binde wird mit der Hand festgehalten und die Binde kann sich nun selbständig durch Loslassen des rollenförmigen Körpers bis zum Erreichen der Abrollsicherung entrollen, wobei die Abrollsicherung ein weiteres Abrollen verhindert. Auf diese Weise wird das Wundkissen freigelegt und kann auf eine zu bedeckende Wunde oder Verletzung aufgelegt werden. Gleichzeitig ist kein Festhalten und Sichern der noch in Rollenform vorliegenden Restbinde notwendig, so dass beide Hände in dieser ersten Phase der Wundversorgung zur Verfügung stehen. Die Binde wird um den Körperbereich, an dem sich die Wunde befindet, herumgewickelt. Mit dem Wundkissen wird auch gleichzeitig der Druckapplikator, der auf der gegenüberliegenden Seite der Binde angeordnet ist, freigelegt. Der Wundapplikator weist dabei im angelegten Zustand nach außen. Die Binde wird nun so weit um den Körperbereich herumgelegt, bis der Bereich des Druckapplikators erreicht wird. Sie wird dann in den Druckapplikator eingehakt, indem die Binde in den Spalt zwischen dem ersten Fixierungselement und der Basisplatte eingeführt wird. Danach wechselt die Wickelrichtung der Binde. Das Einführen erfolgt dabei von der bindenzugewandten Seite des Fixierungselements. D.h., die Binde läuft unter dem Fixierungselement durch, durch den Spalt und unter Richtungswechsel über diesen hinweg. Dabei soll unter dem ersten Fixierungselement das Fixierungselement verstanden werden, das als erstes durch die Binde beim Umschlingen des Körperteils erreicht wird. Nach dem Wechsel der Wickelrichtung wird dann ein weiteres Mal um den betreffenden Körperteil gewickelt, bis wieder der Druckapplikator erreicht wird, jetzt im Bereich des zweiten Fixierungselements und die Binde in den Druckapplikator eingehakt wird, wiederum indem sie in den Spalt zwischen dem zweiten Fixierungselement und der Basisplatte eingeführt wird. Dies erfolgt wie vorstehend beschrieben. Durch Anziehen der Binde wird der benötigte Druck durch den Druckapplikator auf die Wunde ausgeübt. Der Druck wird dabei im Wesentlichen durch die Basisplatte auf die Wunde aufgebracht. Falls die Wundauflage beim Anlegen der Binde verrutscht ist, kann die Lage jetzt noch korrigiert werden. Eine solche Lagekorrektur kann notwendig werden, wenn nach Einhaken der Binde auf der ersten Seite des Druckapplikators z. B. zu viel Zug aufgebracht wird und es zu einem Mitdrehen der Binde am Körperteil kommt. Abschließend wird die Binde nun weiter um den betroffenen Körperteil gewickelt, bis das Ende erreicht ist. Mittels des Befestigungselements, insbesondere eines endständigen Verschlussbügels, wird die angelegte Binde fixiert.

Die Erfindung soll im Folgenden anhand einer Zeichnung näher erläutert werden.

Dabei zeigen:
- Figur 1: eine erste perspektivische Ansicht der erfindungsgemäßen Binde,
- Figur 2: eine weitere Ansicht der erfindungsgemäßen Binde.

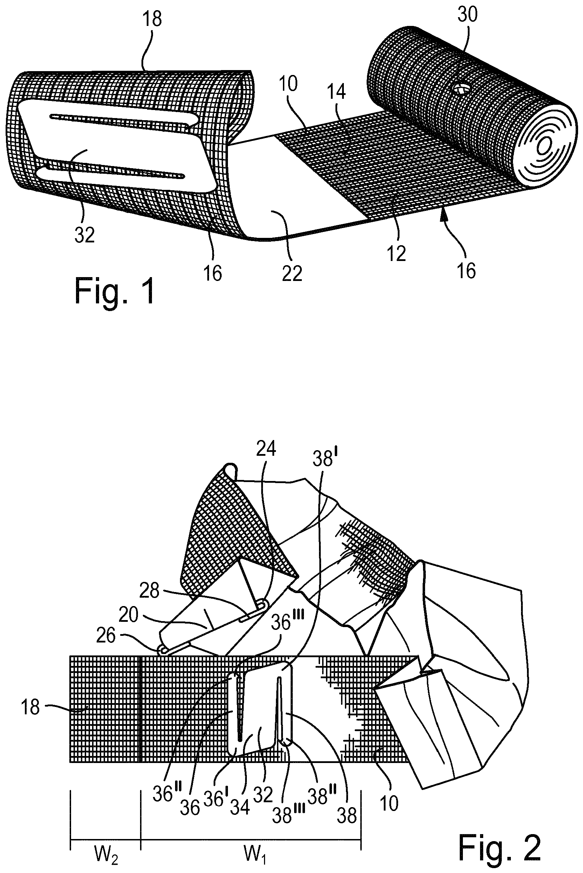

Figur 1 zeigt eine erfindungsgemäße Binde 10, wobei die Rollenform, in der die Binde konfektioniert ist, am rechten Bildrand noch zu erkennen ist. Dabei ist die Binde bis zu einer Abrollsicherung 30 abgerollt. Die Länge des bereits abgerollten Endes beträgt ca. 40 cm. Die Binde weist eine Flachmaterialbahn auf, die mit dem Bezugszeichen 12 gekennzeichnet ist, mit einer ersten Seite 14 sowie einer zweiten Seite 16. Darüber hinaus hat die Flachmaterialbahn zwei Enden 18 und 20, wobei das zweite Ende 20 in Figur 1 sich innerhalb der noch aufgerollten Konfiguration befindet. Die Binde 10 des Ausführungsbeispiels ist monoelastisch, wobei die Wellenform der Flachmaterialbahn im entspannten Zustand sowohl in Figur 1 als auch in Figur 2 zu erkennen ist, die sich dadurch ergibt, dass die elastischen Kettfäden während des Webprozesses gedehnt sind und sich danach entspannen. Die Dehnbarkeit liegt bei ca. 200%. Auf der ersten Seite 14 der Binde 10 ist ein Wundkissen 22 angeordnet, bei dem ein absorbierendes Material in einer inneren Umhüllung vorgesehen ist und diese innere Umhüllung durch zwei Vlieslagen als Hülllagen einer Umhüllung umschlossen ist, wobei die Vlieslagen so gestaltet sind, dass ein Flüssigkeitszutritt von einer Wunde in das Wundkissen ermöglicht ist. Weiterhin kann vorgesehen sein, dass die wundabgewandte Seite des Wundkissens 22 flüssigkeitsundurchlässig oder zumindest flüssigkeitsabweisend ausgebildet ist.

Das Wundkissen 22 erstreckt sich dabei vorzugsweise über die gesamte Breite der Binde 10. Das Wundkissen 22 kann klebend oder mittels Nähen an der Binde 10 festgelegt sein. Die Länge des Wundkissens ist in Figur 2 mit dem Bezugszeichen W₁ gekennzeichnet.

Am ersten Ende 18 der Binde ist eine Art Anfassabschnitt dadurch gestaltet, dass ein Teil der Binde auf sich selbst umgeschlagen und dort vernäht wird. Dieser Anfassabschnitt hat eine Länge W₂, die in etwa 7 cm beträgt. Hierdurch wird das erste Ende 18 stabilisiert und kann besser gehandhabt werden. Das Wundkissen besitzt eine Erstreckung von ca. 10 x 20 cm und die Binde weist eine Breite von ca. 10 cm auf. Am zweiten Ende 20 ist ein Befestigungselement 24 vorgesehen, das hier durch einen Verschlussbügel 26 gebildet ist, der bei fertig an einem Patienten angelegter Binde 10 unter weiter unten liegenden Wicklungen mit seinen zwei Bügelelementen 28 gehakt werden kann. Um einen Abbindeeffekt zu erreichen, kann mit Hilfe des Verschlussbügels durch Drehen desselben um eine Achse senkrecht zur Bindenebene der Druck in bekannter Weise auf eine Wunde weiter erhöht werden.

Um zu verhindern, dass sich die Binde mehr als gewünscht abrollt, während das Wundkissen 22 auf einer Wunde platziert wird, ist eine Abrollsicherung 30 vorgesehen, die durch einen Faden gebildet ist, der eine der Bindenwicklungen mit einer darunter liegenden Wicklung verbindet und so ein weiteres Abrollen verhindert. Ist die erste Fixierung der Wundauflage erfolgt und soll die Binde vollständig an der Person festgelegt werden, kann die Abrollsicherung gelöst bzw. im vorliegenden Fall durch Zerreißen des Fadens zerstört werden. Die Abrollsicherung 30 ist dabei vorzugsweise so angebracht, dass das Wundkissen 22 auf einer Wunde platziert werden kann und die Binde 10 dann einmal um den zu behandelnden Körperteil herumgelegt werden kann, so dass die Binde 10 dann in einem Druckapplikator 32 festgelegt werden kann. Die Festlegung erfolgt dabei, indem der Druckapplikator 32 aus einer Basisplatte 34 besteht, mit der der Druckapplikator 32 an der Binde 10 festgelegt ist, sowie zwei Fixierungselementen 36 und 38, die in Längsrichtung auf beiden Seiten der Basisplatte 34 vorgesehen sind. Die Basisplatte 34 weist dabei im vorliegenden Ausführungsbeispiel eine parallelogrammartige Form auf und besteht, ebenso wie die Fixierungselemente 38, 38, die einstückig hiermit verbunden sind, aus einem Polycarbonat. Die Fixierungselemente 36, 38 schließen zwischen sich und der Basisplatte 34 einen Spalt ein, wobei der Spalt entweder im Wesentlichen über die gesamte Länge gleich ausgebildet sein kann oder, wie hier vorliegend, sich V-förmig verbreitern kann. Die Fixierungselemente 36, 38 sind mit einem Ende 36' bzw. 38' mit der Basisplatte 34 einstückig verbunden, wobei sich die Enden 36' und 38' im Wesentlichen diagonal gegenüberliegen. Die anderen Enden 36" und 38" sind frei, so dass die Binde in den Spalt zwischen der Basisplatte 34 und den Fixierungselementen 36, 38 eingeführt werden kann.

Um ein ungewolltes Lösen der Binde aus dem Spalt zu verhindern, sind an den freien Enden 36", 38" Nasen 36‴ bzw. 38‴ vorgesehen, die die Binde 10 im Spalt sichern.

Die Binde 10 wird nun beim Anlegen zunächst bis zur Abrollsicherung 30 abgerollt und das Wundkissen 22 wird auf der Verletzung platziert. Die Binde 10 wird dann um den betroffenen Körperteil herumgelegt und in den ersten Spalt zwischen dem ersten Fixierungselement 36 und der Basisplatte 34 eingeschoben. Hierdurch wird eine erste Fixierung erreicht. Danach wird die Wickelrichtung geändert und die Binde 10 wird unter Zerstörung des Fadens, der die Abrollsicherung bildet, um den betroffenen Körperteil wiederum herumgelegt bis zum zweiten Fixierungselement 38 und dort in den Spalt zwischen Fixierungselement 38 und Basisplatte 34 eingeführt. Danach kann durch Spannen ein Druck auf die Wunde über die Basisplatte 34 ausgeübt werden, wobei dieser Druck durch entsprechendes Anziehen erreicht wird. Durch die zweimalige Umlenkung der Wickelrichtung, die nochmals nach Einhaken hinter das Fixierungselement 38 geändert wird, wird eine sehr gleichmäßige Druckaufbringung erreicht. Danach wird die Binde 10 vollständig an der betroffenen Person festgewickelt und die Binde 10 wird über das Befestigungselement 26 endfixiert.

Auf die vorstehend beschriebene Weise kann besonders einfach eine gleichmäßige Krafteinleitung und Applikation einer Binde auch in Krisensituationen erfolgen.

## Patentansprüche

1. Binde (10) umfassend
e. eine Flachmaterialbahn (12) mit einer ersten (14) und einer zweiten (16) Seite und einem ersten (18) und zweiten (20) in Längsrichtung der Binde (10) beabstandeten Ende,
f. ein Wundkissen (22) auf der ersten Seite der Flachmaterialbahn (12),
g. einen Druckapplikator (32) auf der zweiten Seite (16) der Flachmaterialbahn (12) gegenüber dem Wundkissen (22),
h. ein Befestigungselement (24) am zweiten Ende (20) der Flachmaterialbahn (12) zur Befestigung des zweiten Endes (20) der Flachmaterialbahn (12) auf oder an sich selber bei an einem Träger angelegter Binde (10), **dadurch gekennzeichnet, dass**
der Druckapplikator (32) eine Basisplatte (34) aufweist und zwei in Längsrichtung der Binde (10) beidseits der Basisplatte (34) angeordnete längliche Fixierungselemente (36, 38), die sich in einem Winkel, insbesondere senkrecht, zur Längsrichtung der Binde (10) erstrecken, wobei jeweils das eine Ende (36', 38') der Fixierungselemente (36, 38) an der Basisplatte (34) festgelegt ist und das andere Ende (36", 38") frei ist und die Fixierungselemente (36, 38) über ihre restliche Länge zwischen sich und der Basisplatte (34) einen Spalt ausbilden.

2. Binde nach Anspruch 1, **dadurch gekennzeichnet, dass** die an der Basisplatte (34) festgelegten Enden (36', 38') der Fixierungselemente (36, 38)einander diagonal gegenüberliegen.

3. Binde nach Anspruch 1 oder 2, **dadurch gekennzeichnet, dass** die Basisplatte (34) eine rechteckige oder parallelogrammartige Form aufweist.

4. Binde nach einem der vorherigen Ansprüche, **dadurch gekennzeichnet, dass** die freien Enden (36", 38") der Fixierungselemente (36, 38) insbesondere quer zur Längsrichtung der Binde (10) über die Basisplatte (34) hinausragen.

5. Binde nach einem der vorherigen Ansprüche, **dadurch gekennzeichnet, dass** der Druckapplikator (32) mittig über dem Wundkissen (22) angeordnet ist.

6. Binde nach einem der vorherigen Ansprüche, **dadurch gekennzeichnet, dass** am ersten Ende (18) der Flachmaterialbahn (12) ein Anfassabschnitt ausgebildet ist, insbesondere durch Umschlagen der Flachmaterialbahn (12) in diesem Bereich auf sich selber und Festlegen.

7. Binde nach einem der vorherigen Ansprüche, **dadurch gekennzeichnet, dass** der Spalt zwischen den Fixierungselementen (36, 38) und der Basisplatte im Wesentlichen gerade verläuft.

8. Binde nach einem der vorherigen Ansprüche, **dadurch gekennzeichnet, dass** die Fixierungselementen (36, 38) und/oder die Basisplatte (34) wenigstens abschnittsweise elastisch verformbar sind.

9. Binde nach einem der vorherigen Ansprüche, **dadurch gekennzeichnet, dass** das Flachbahnmaterial (12) aus einem monoelastischen Gewebe besteht.

10. Binde nach einem der vorherigen Ansprüche, **dadurch gekennzeichnet, dass** die Binde (10) eine Kompressionsbinde ist.

11. Binde nach einem der vorherigen Ansprüche, **dadurch gekennzeichnet, dass** die Binde (10) in Rollenform konfektioniert ist, so dass das erste Ende (38) der Flachmaterialbahn (12) außenliegend ist.

12. Binde nach Anspruch 11, **dadurch gekennzeichnet, dass** eine lösbare und/oder zerstörbare Abrollsicherung vorgesehen ist, die nach einer definierten Länge ausgehend vom ersten Ende (18) ein weiteres Abrollen verhindert.

13. Binde nach einem der Ansprüche 6 bis 12, **dadurch gekennzeichnet, dass** das Wundkissen (22) sich in Längsrichtung der Binde an den Anfassabschnitt anschließt.

14. Binde nach einem der vorherigen Ansprüche, **dadurch gekennzeichnet, dass** das Wundkissen eine Umhüllung aufweist, die mit einem absorbierenden Material, insbesondere einem Cellulosematerial, einem SAP-Material und/oder einem Alginat, insbesondere einem Cellulosefluffmaterial gefüllt ist.

## Claims

1. A bandage (10) including
e. a flat material web (12) having a first (14) and a second (16) side and a first (18) and second (20) end which are spaced apart in the longitudinal direction of the bandage (10),
f. a wound dressing pad (22) on the first side of the flat material web (12),
g. a pressure applicator (32) on the second side (16) of the flat material web (12) opposite the wound dressing pad (22),
h. a fastening element (24) at the second end (20) of the flat material web (12) for fastening the second end (20) of the flat material web (12) on or to itself when the bandage (10) is positioned on a carrier, **characterized in that**
the pressure applicator (32) comprises a base plate (34) and two elongated fixing elements (36, 38) which are arranged in the longitudinal direction of the bandage (10) on both sides of the base plate (34) and extend at an angle, in particular perpendicularly, to the longitudinal direction of the bandage (10), wherein in each case the one end (36', 38') of the fixing elements (36, 38) is secured to the base plate (34) and the other end (36", 38") is free and the fixing elements (36, 38) realize a gap between them and the base plate (34) over their remaining length.

2. The bandage as claimed in claim 1, **characterized in that** the ends (36', 38') of the fixing elements (36, 38) fixed to the base plate (34) are situated diagonally opposite one another.

3. The bandage as claimed in claim 1 or 2, **characterized in that** the base plate (34) comprises a rectangular or parallelogram-like form.

4. The bandage as claimed in one of the preceding claims, **characterized in that** the free ends (36", 38") of the fixing elements (36, 38) project beyond the base plate (34), in particular transversely with respect to the longitudinal direction of the bandage (10).

5. The bandage as claimed in one of the preceding claims, **characterized in that** the pressure applicator (32) is arranged centrally above the wound dressing pad (22).

6. The bandage as claimed in one of the preceding claims, **characterized in that** a holding portion is realized at the first end (18) of the flat material web (12), in particular by folding the flat material web (12) over on itself in said region and securing it.

7. The bandage as claimed in one of the preceding claims, **characterized in that** the gap between the fixing elements (36, 38) and the base plate extends in a substantially straight manner.

8. The bandage as claimed in one of the preceding claims, **characterized in that** the fixing elements (36, 38) and/or the base plate (34) are deformable elastically at least in portions.

9. The bandage as claimed in one of the preceding claims, **characterized in that** the flat material web (12) consists of a mono-elastic woven fabric.

10. The bandage as claimed in one of the preceding claims, **characterized in that** the bandage (10) is a compression bandage.

11. The bandage as claimed in one of the preceding claims, **characterized in that** the bandage (10) is made up in roll form so that the first end (38) of the flat material web (12) is on the outside.

12. The bandage as claimed in claim 11, **characterized in that** a releasable and/or destructible unroll lock is provided which prevents further unrolling after a defined length proceeding from the first end (18).

13. The bandage as claimed in one of claims 6 to 12, **characterized in that** the wound dressing pad (22) connects to the holding portion in the longitudinal direction of the bandage.

14. The bandage as claimed in one of the preceding claims, **characterized in that** the wound dressing pad comprises an envelope which is filled with an absorbent material, in particular a cellulose material, a super-absorbent polymer (SAP) material and/or an alginate, in particular a cellulose fluff material.

## Revendications

1. Bandage (10) comprenant
e. une bande de matériau plat (12) présentant un premier (14) et un deuxième (16) côtés et une première (18) et une deuxième (20) extrémités espacées dans la direction longitudinale du bandage (10),
f. un coussin vulnéraire (22) sur le premier côté de la bande de matériau plat (12),
g. un applicateur de pression (32) sur le deuxième côté (16) de la bande de matériau plat (12) à l'opposé du coussin vulnéraire (22),
h. un élément de fixation (24) à la deuxième extrémité (20) de la bande de matériau plat (12) pour fixer la deuxième extrémité (20) de la bande de matériau plat (12) sur ou à elle-même lorsque le bandage (10) est placé sur un porteur, **caractérisé par le fait que**
l'applicateur de pression (32) présente une plaque de base (34) et deux éléments de fixation (36, 38) allongés qui sont disposés dans la direction longitudinale du bandage (10) de part et d'autre de la plaque de base (34) et s'étendent à un angle, en particulier perpendiculairement, à la direction longitudinale du bandage (10), dans lequel respectivement l'une (36', 38') des extrémités des éléments de fixation (36, 38) est fixée à la plaque de base (34) et l'autre extrémité (36", 38") est libre et les éléments de fixation (36, 38) forment une fente entre eux et la plaque de base (34) sur leur longueur restante.

2. Bandage selon la revendication 1, **caractérisé par le fait que** les extrémités (36', 38') des éléments de fixation (36, 38) qui sont fixées à la plaque de base (34) sont diagonalement opposées l'une à l'autre.

3. Bandage selon la revendication 1 ou 2, **caractérisé par le fait que** la plaque de base (34) présente une forme rectangulaire ou de type parallélogramme.

4. Bandage selon l'une quelconque des revendications précédentes, **caractérisé par le fait que** les extrémités libres (36'', 38'') des éléments de fixation (36, 38) dépassent la plaque de base (34) en particulier transversalement à la direction longitudinale du bandage (10) .

5. Bandage selon l'une quelconque des revendications précédentes, **caractérisé par le fait que** l'applicateur de pression (32) est disposé de manière centrale au-dessus du coussin vulnéraire (22).

6. Bandage selon l'une quelconque des revendications précédentes, **caractérisé par le fait qu'**une section de préhension est formée à la première extrémité (18) de la bande de matériau plat (12), en particulier en repliant la bande de matériau plat (12) dans cette zone sur elle-même et en la fixant.

7. Bandage selon l'une quelconque des revendications précédentes, **caractérisé par le fait que** la fente entre les éléments de fixation (36, 38) et la plaque de base s'étend pour l'essentiel de manière droite.

8. Bandage selon l'une quelconque des revendications précédentes, **caractérisé par le fait que** les éléments de fixation (36, 38) et/ou la plaque de base (34) sont élastiquement déformables au moins par sections.

9. Bandage selon l'une quelconque des revendications précédentes, **caractérisé par le fait que** le matériau de bande plat (12) est constitué d'un tissu monoélastique.

10. Bandage selon l'une quelconque des revendications précédentes, **caractérisé par le fait que** le bandage (10) est un bandage de compression.

11. Bandage selon l'une quelconque des revendications précédentes, **caractérisé par le fait que** le bandage (10) est confectionné sous forme de rouleau de sorte que la première extrémité (38) de la bande de matériau plat (12) est située à l'extérieur.

12. Bandage selon la revendication 11, **caractérisé par le fait qu'**il est prévu un dispositif anti-déroulement détachable et/ou destructible qui empêche un autre déroulement après une longueur définie à partir de la première extrémité (18).

13. Bandage selon l'une quelconque des revendications 6 à 12, **caractérisé par le fait que** le coussin vulnéraire (22) est contigu, dans la direction longitudinale du bandage, à ladite section de préhension.

14. Bandage selon l'une quelconque des revendications précédentes, **caractérisé par le fait que** le coussin vulnéraire présente une enveloppe qui est remplie d'un matériau absorbant, en particulier d'un matériau cellulosique, d'un matériau SAP et/ou d'un alginate, en particulier d'un matériau duvet cellulosique.
